# EUROPÄISCHE PATENTSCHRIFT

(11) **EP 1 829 829 B1**
(45) Veröffentlichungstag und Bekanntmachung des Hinweises auf die Patenterteilung: **04.04.2012**
(21) Anmeldenummer: 07004434.2
(22) Anmeldetag: 05.03.2007
(51) Int. Cl.: C02F 11/12

(54) **Verfahren zur Trennung von Biomasse**
Method for separating biomass
Procédé de séparation de biomasse

(30) Priorität: 03.03.2006 DE 102006010449
(43) Veröffentlichungstag der Anmeldung: 05.09.2007
(73) Patentinhaber: GETproject GmbH & Co. KG, 24109 Kiel (DE)
(72) Erfinder: Götz, Johann, 24105 Kiel (DE)
(74) Vertreter: Lobemeier, Martin Landolf

(56) Entgegenhaltungen:
- WO-A-92/15540
- DE-C1- 19 719 895
- JP-A- 4 326 994
- JP-A- 2002 233 900
- JP-A- 2005 329 396
- US-B1- 6 447 687

## Beschreibung

Die Erfindung betrifft eine Vorrichtung zur Trennung von Biomasse in einen Feststoffanteil und eine unter Erzeugung von Biogas zu vergärende flüssige Phase.

Die Verwertung von Energiepflanzen erfolgt üblicherweise in Biogasanlagen: Nach der Vergärung der gehäckselten und silierten Biomassen, der so genannten Ganzpflanzenvergärung, wird mit dem so gewonnenen Biogas mit Hilfe von Gasmotoren elektrische Energie erzeugt. Nachteilig bei dem Verfahren ist, dass die vergärbaren Pflanzeninhaltsstoffe in eine nur bedingt vergärbare Zellstruktur aus Zellulose und Lignin eingeschlossen ist. Für eine ausreichende Energieausbeute ist daher eine lange Verweilzeiten im Fermenter erforderlich. Zudem sind massive Rührwerke erforderlich, die mit erheblichem Energieaufwand für eine Durchmischung des Fermenter-Inhalts sorgen und die Bildung von Schwimmschichten verhindern.

Bekannt ist eine Anlage zur Erzeugung von Biogas aus silierten Energiepflanzen, bei der die Biomasse durch eine mechanische Entwässerung mit Hilfe von Pressen in einen Feststoff, der zu einem großen Anteil aus der Zellstruktur der Pflanzen besteht, und eine flüssigen Phase mit gelösten und ungelösten vergärbaren Pflanzenbestandteilen sowie anorganischen Pflanzeninhaltsstoffen getrennt wird. Nur die Flüssigphase wird der Fermentation zugeführt. Im Vergleich zur Ganzpflanzenvergärung erfolgt deren Vergärung mit sehr kurzen Verweilzeiten. Außerdem werden keine Rührwerke benötigt. Notwendig ist lediglich eine gelegentliche Umwälzung der Fermenterinhalte, um Sinkstoffe wieder in Suspension zu bringen. Die Feststoffe werden bei dem bekannten Verfahren getrocknet und z. B. zu Brennstoffen verarbeitet.

Im Stand der Technik sind feucht-saure Aufschlußverfahren nach der DE 195 19 213 A1, Destillationsverfahren nach der DE 44 02 559 A1 und einfache Entwässerungsverfahren nach der DE 320 375 C bekannt.

Darüber hinaus sind beispielsweise aus der JP 04 326 994 A, JP 2002 233 900 A, JP 2005 329 396 A und der WO 92/15540 A1 bereits Vorrichtungen bekannt, bei denen Biomasse in eine Presse eingebracht und das daraus abgezogene Wasser einem Fermenter zugeführt wird.

Der Erfindung liegt die Aufgabe zugrunde, ein Verfahren zu schaffen, bei dem einerseits die leicht vergärbaren Pflanzenanteile möglichst vollständig in die flüssige Phase überführt und der Vergärung für die Biogasproduktion zugeführt werden, andererseits aber auch die anorganischen Pflanzeninhaltsstoffe möglichst vollständig aus dem Feststoff entfernt werden, die bei einer Verbrennung zu einer unerwünschten Absenkung des Ascheschmelzpunktes und zu Korrosionserscheinungen führen. Außerdem ist anzustreben, dass die anorganischen Pflanzeninhaltsstoffe zusammen mit dem Gärrest wieder auf die Anbauflächen der Pflanzen ausgebracht werden.

Zur Lösung dieser Aufgabe wird ein Verfahren mit den Merkmalen des Anspruchs 1 vorgeschlagen.

Bei dem vorgeschlagenen Verfahren wird die Biomasse in einem geeigneten Behälter mit einer Prozessflüssigkeit, dem Frischwasser beigemischt sein kann, vermischt und verrührt. Bei dem Behälter handelt es sich entweder um einen vertikalen Rührbehälter, einer horizontalen Trommel, einer Mischschnecke oder einer vergleichbaren Einrichtung.

Die durch das Abpressen der konditionierten Biomassen gewonnene Suspension wird nach einer Abkühlung auf die geeignete Prozesstemperatur einer Fermentation zugeführt. Die dabei zurückgewonnene Wärme kann zur Trocknung der abgetrennten Feststoffe eingesetzt werden.

Die nach dem Abpressen in dem Feststoff enthaltene Wärme-Energie wird in sofern ebenfalls zurückgewonnen, als das der Biomasse entsprechend weniger Energie für die Trocknung zugeführt werden muss.

In der nachfolgenden Figur ist ein Ausführungsbeispiel einer derartigen Anlage dargestellt.

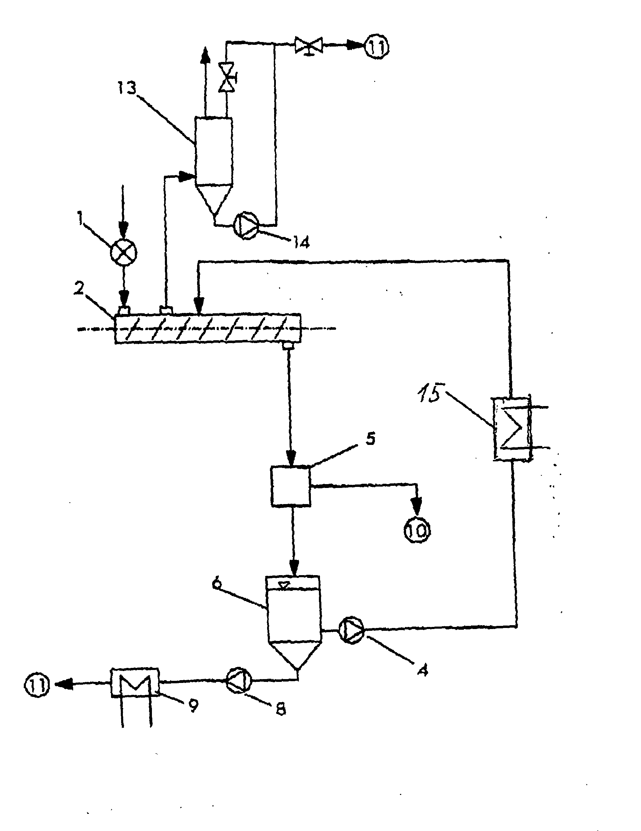

Die Figur zeigt einen mit einer Zellenradschleuse 1 versehenen Mischbehälter 2, in den zum einen die Biomasse und zum anderen eine Prozessflüssigkeit dosiert eingebracht wird. In dem Mischbehälter 2 wird die Biomasse von einem Rührwerk durchmischt, wobei der Wassergehalt der Biomasse erhöht wird.

Die aus dem Mischbehälter 2 ausgetragene, aufbereitete Biomasse wird einer Presse 5 zugeführt, in der eine Trennung in einen Feststoffteil und eine flüssige Phase erfolgt.

Durch den erhöhten Wassergehalt der Biomasse und den dadurch bei dem Abpressen erhöhten Wasserfluss aus der Zellstruktur der Biomasse heraus werden gut vergärbare Pflanzenanteile und mineralische Inhaltsstoffe weitgehend in die Flüssigkeitsphase überführt.

Die flüssige Phase wird einem Absetzbehälter 6 zugeführt, die um Schwebstoffe abgereicherte Flüssigkeit wird von einer Dosierpumpe 4 durch einen von der Abwärme des mit dem durch die Vergärung der flüssigen Phase gewonnen Biogas betriebenen Gasmotors gespeisten Wärmetauscher 15 als Prozessflüssigkeit dem Mischbehälter 2 zugeführt.

Der mit Feststoffen angereicherte Teil der flüssigen Phase wird der Vergärung 11 für die Biogasproduktion zugeführt, wobei in einem Wärmetauscher 9 die Temperatur auf die Prozesstemperatur des Vergärungsprozesses 11 reduziert wird (die dabei rückgewonnene Wärme kann zur Trocknung des Feststoffteils genutzt werden).

Der Mischbehälter 2 ist bei dem dargestellten Ausführungsbeispiel mit einem Wäscher 13 zur Rückgewinnung von flüchtigen organischen Bestandteilen der Biomasse ausgerüstet werden. Mit der Umwälzpumpe 14 wird das Waschwasser nach Anreicherung mit Pflanzeninhaltsstoffen ebenfalls dem Vergärungsprozess 11 zugeführt.

Die hier vorgeschlagene Anlage zur Trennung von Biomasse in einen trockenen gepressten Feststoffanteil und zu vergärende flüssige Phase ermöglicht die Erzeugung marktgängiger speicherbarer Energieträger aus nachwachsenden Rohstoffen, besitzt dabei aber den entscheidenden Vorteil, dass die bei der üblichen Verwertung des Biogases zur Stromerzeugung in den Gasmotoren anfallenden Abwärme, soweit sie nicht als Prozess benötigt wird, vollständig für die Trocknung des Feststoffanteils genutzt werden kann.

Das Problem, dass die Abwärme der Gasmotoren nicht vollständig genutzt werden kann, entfällt also. Durch die annähernd vollständige Entfernung der anorganischen Pflanzenbestandteile aus den Feststoffen wird die Qualität der daraus produzierten Brennstoffe ganz erheblich verbessert.

Auch die mechanische Entwässerung der Biomasse wird verbessert, so dass niedrigere Restfeuchten des Feststoffes erreicht werden.

Der Aufschluss erfolgt mit erhitzter Prozessflüssigkeit. Der Mischbehälter 2 kann zusätzlich Beheizung über Wärmeaustauschflächen oder durch direkte Beschickung mit Wasserdampf oder in Kombination beheizt werden. Für die Beheizung mit Wasserdampf wird eine Temperatur zwischen 150 und 220 °C vorgeschlagen, insbesondere 170 - 205 °C.

Der Aufschluss wird bisher bevorzugt in einer Mischschnecke durchgeführt und kann aber auch in einer Trommel oder in einem Rührbehälter erfolgen. Die Beheizung kann in allen Fällen alternativ direkt oder indirekt oder in Kombination davon erfolgen.

Weiter ist die Bewirkung eines Überdrucks in dem Mischbehälter 2 zwischen 1,5 und 2 für den Aufschluss der Biomasse hilfreich.

Die mechanische Entwässerung und Abtrennung der mobilisierten vergärbaren Inhaltsstoffe erfolgt dagegen bevorzugt unter Temperaturen zwischen 45 und 90 °C in einer Zentrifuge oder einer Walzenpresse.

Wenn der Biomassen vergorenes Substrat aus dem Vergärprozess beigegeben wird, verbessert sich die Ausbeute.

Schließlich sollten die entstehenden flüchtigen organischen Stoffe einem Abluftwäscher zugeführt werden, und dort eine Abscheidung der organischen Stoffe mit anschließender Zufuhr in den Vergärprozess erfolgen.

Durch die Vorrichtung zur mechanischer Trennung von Biomassen nach einem Aufbereiten mit erhitzter Prozessflüssigkeit in einen Feststoffanteil, im wesentlichen bestehend aus der Zellstruktur aus Zellulose und Lignin und eine flüssigen Phase mit den vergärbaren Pflanzenanteilen der Gruppe Stärke, Eiweiß, Zucker, Öle und Fette sowie den anorganischen Pflanzeninhaltsstoffe erfolgt ein schneller und stabiler Vergärprozess mit wenig Rückständen, so dass eine Feststoff erzeugt wird, der gute Verbrennungseigenschaften hat, der aber auch als Dünger ausgebracht werden kann.

## Patentansprüche

1. Verfahren zur Trennung von Biomasse in einen Feststoffanteil und eine flüssige Phase, mit den Schritten:
- Einbringen der Biomasse in einen Mischbehälter (2),
- Vermischen der Biomasse in dem Mischbehälter (2) mit einer Prozessflüssigkeit,
- Abführen der mit der Prozessflüssigkeit vermischten Biomasse aus dem Mischbehälter (2) in eine Presse (5),
- Trennen der aus dem Mischbehälter (2) abgeführten aufbereiteten Biomasse in einen Feststoffanteil und einen um die im Mischbehälter (2) mit den gut vergärbaren organischen und der anorganischen Pflanzeninhaltsstoffe angereicherte flüssige Phase in der Presse (5), und
- Zuführen des Feststoffanteils zu einer Trocknung (10),
**dadurch gekennzeichnet, dass**
- zur Erzeugung von Biogas durch Vergären der flüssigen Phase - beim Vermischen der Biomasse der Wassergehalt der Biomasse erhöht wird,
es dem mit Schwebstoffen angereicherten Teil der flüssigen Phase erlaubt wird, sich in einem Absetzbehälter (6) abzusetzen
die von den Schwebstoffenentreicherte flüssige Phase von einer Dosierpumpe (4) durch einen Wärmetauscher (15) und nach Erwärmung der von den Schwebstoffen entreicherten flüssigen Phase im Wärmetauscher (15) als Prozessflüssigkeit in den Mischbehälter (2) rückgeführt wird, und
der in dem Absetzbehälter (6) mit den Feststoffen angereicherte Teil der flüssigen Phase einer Fermentation (11) zugeführt wird.

## Claims

1. A method for separating biomass into a solid fraction and a liquid phase, having the steps:
- introducing the biomass into a mixing tank (2),
- mixing the biomass in the mixing tank (2) with a process liquid,
- discharging the biomass mixed with the process liquid from the mixing tank (2) into a press (5),
- separating, in the press (5), the conditioned biomass that has been discharged from the mixing tank (2) into a solid fraction and a liquid phase, enriched in the mixing tank (2), with the organic plant ingredients that can be fermented well and the inorganic plant ingredients, and
- subjecting the solid fraction to a drying process (10),
**characterized in that**
- to produce biogas by fermenting the liquid phase -
the water content of the biomass is increased when mixing the biomass,
that part of the liquid phase that is enriched with suspended particles is permitted to settle in a settling tank (6),
the liquid phase with suspended particles removed is fed back by a metering pump (4) through a heat exchanger (15) and after heating the liquid phase with suspended particles removed in the heat exchanger (15), as process liquid into the mixing tank (2), and
the part of the liquid phase that is enriched with the solids in the settling tank (6) is subjected to a fermentation (11).

## Revendications

1. Procédé pour la séparation de la biomasse en une partie solide et en une phase liquide en respectant les étapes suivantes :
- Déposer la biomasse dans un mélangeur (2),
- Mélanger la biomasse dans le mélangeur (2) avec un fluide de processus,
- Faire couler la biomasse mélangée au fluide de processus du mélangeur (2) dans une presse hydraulique (5),
- Séparer dans la presse hydraulique (5) la biomasse préparée extraite du mélangeur (2) en une partie solide et en une phase liquide enrichie de composants végétaux fermentes cibles organiques et anorganiques dans le mélangeur (2) et
- Transport de la partie solide vers un séchage (10)
considérant que
- pour produire du biogaz en fermentant la phase liquide,
la teneur en eau de la biomasse est augmentée lors du mélange de la biomasse,
la partie enrichie de matières en suspension de la phase liquide peut se déposer dans un bassin de décantation (6),
la phase liquide débarrassée des matières en suspension est à nouveau transportée dans le mélangeur (2) sous forme de fluide de processus par une pompe de dosage (4) et au moyen d'un échangeur thermique (15) après chauffage de la phase liquide débarrassée des matières en suspension dans l'échangeur thermique (15)
et la partie enrichie par les matières solides de la phase liquide dans le bassin de décantation (6) est soumise à fermentation (11)
